# EUROPEAN PATENT SPECIFICATION

(11) **EP 3 363 802 B1**
(45) Date of publication and mention of the grant of the patent: **20.11.2019**
(21) Application number: 17156518.7
(22) Date of filing: 16.02.2017
(51) Int. Cl.: C07D 498/14, A61K 31/4985, A61P 31/18

(54) **CRYSTALLINE FORM OF CABOTEGRAVIR SODIUM**
KRYSTALLINE FORM VON CABOTEGRAVIR NATRIUMSALZ
FORME CRISTALLINE DU SEL DE SODIUM DE CABOTEGRAVIR

(43) Date of publication of application: 22.08.2018
(73) Proprietor: Sandoz AG, 4056 Basel (CH)
(72) Inventor: ADAMER, Verena, 6250 Kundl (AT); THALER, Andrea, 6250 Kundl (AT)
(74) Representative: Prüfer & Partner mbB Patentanwälte · Rechtsanwälte

(56) References cited:
- EP-A1- 3 045 206
- WO-A1-2010/068253
- WO-A1-2011/119566
- WO-A1-2015/177537
- BRIAN A. JOHNS ET AL: "Carbamoyl Pyridone HIV-1 Integrase Inhibitors 3. A Diastereomeric Approach to Chiral Nonracemic Tricyclic Ring Systems and the Discovery of Dolutegravir (S/GSK1349572) and (S/GSK1265744)", JOURNAL OF MEDICINAL CHEMISTRY, vol. 56, no. 14, 25 July 2013 (2013-07-25) , pages 5901-5916, XP055138762, ISSN: 0022-2623, DOI: 10.1021/jm400645w

## Description

### FIELD OF THE INVENTION

The present invention relates to a crystalline form of cabotegravir sodium and to a process for its preparation. Furthermore, the invention relates to a pharmaceutical composition comprising said crystalline form of cabotegravir sodium, preferably in a predetermined and/or effective amount, and at least one pharmaceutically acceptable excipient. The pharmaceutical composition of the present invention can be used as a medicament, in particular for the treatment and/or prophylaxis of viral infections such as HIV infections.

### BACKGROUND OF THE INVENTION

Cabotegravir is a human immunodeficiency virus type 1 (HIV-1) integrase strand transfer inhibitor (INSTI) currently under development for the treatment of HIV-1 infection in combination with other antiretroviral agents. It is chemically designated as (3*S*,11a*R*)-*N*-[2,4-Difluorophenyl)methyl]-6-hydroxy-3-methyl-5,7-dioxo-2,3,5,7,11,11a-hexahydro[1,3]oxazolo[3,2-*a*]pyrido[1,2-*d*]pyrazine-8-carboxamide and can be represented by the following chemical structure according to Formula (I)

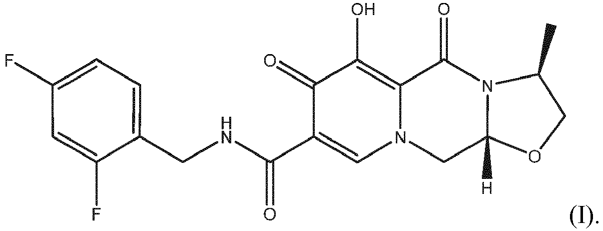

A process for the preparation of cabotegravir sodium is disclosed in Example Z-9 of WO 2006/116764 A1, where cabotegravir was treated with 1 *N* sodium hydroxide in ethanol followed by ether addition. In Example Ae of WO 2010/068253 A1 cabotegravir is dissolved in aqueous ethanol and reacted with 1 *N* aqueous sodium hydroxide solution at 75 °C to the corresponding sodium salt. Cooling the solution followed by filtration, washing with ethanol and drying provided cabotegravir sodium as crystals. In Johns B. A. et. al Carbamoyl Pyridone HIV-1 Integrase Inhibitors 3. A Diastereomeric Approach to Chiral Nonracemic Tricyclic Ring Systems and the Discovery of Dolutegravir (S/GSK1349572) and (S/GSK1265744). J. Med. Chem. 2013, 56, 5901-5916 cabotegravir was treated with 1 *N* aqueous sodium hydroxide in ethanol to obtain cabotegravir sodium as a white solid. In Example 17 of WO 2015/177537 A1 cabotegravir sodium was prepared by treating cabotegravir with 2 *N* aqueous sodium hydroxide in methanol.

The inventors of the present invention repeated the above listed prior art examples (see Reference Examples 1 to 4 herein). According to powder X-ray diffraction the same crystalline form of cabotegravir sodium was obtained in all cases. This form is designated as "Form A" herein.

Different solid state forms of an active pharmaceutical ingredient often possess different properties. Differences in the physicochemical properties of solid state forms can be important for the improvement of pharmaceutical compositions, for example, pharmaceutical formulations with improved dissolution profile or with improved stability or shelf-life can become accessible due to an improved solid state form of an active pharmaceutical ingredient. Also processing or handling of the active pharmaceutical ingredient during the formulation process may be improved. New solid state forms of an active pharmaceutical ingredient can thus have desirable processing properties. They can be easier to handle, better suited for storage, and/or allow for better purification, compared to previously known solid state forms.

Furthermore, the sudden appearance or disappearance of a metastable polymorph can present a problem in pharmaceutical development. Similarly, serious pharmaceutical consequences arise if transformation occurs in a dosage form, e.g. upon storage. It is therefore desirable to use a stable polymorph of an active pharmaceutical ingredient for the preparation of a drug product. Hence, there is a strong need for the provision of a solid form of cabotegravir sodium which is thermodynamically stable and does not undergo phase transformation during pharmaceutical processing or storage. There is also a strong need for the provision of a pharmaceutical composition comprising a solid form of cabotegravir sodium, which is stable at the conditions typically encountered during pharmaceutical processing and storage. The thermodynamically most stable solid form in particular can ensure reliable efficacy and safety for the whole duration of a pharmaceutical composition's shelf-life.

### SUMMARY OF THE INVENTION

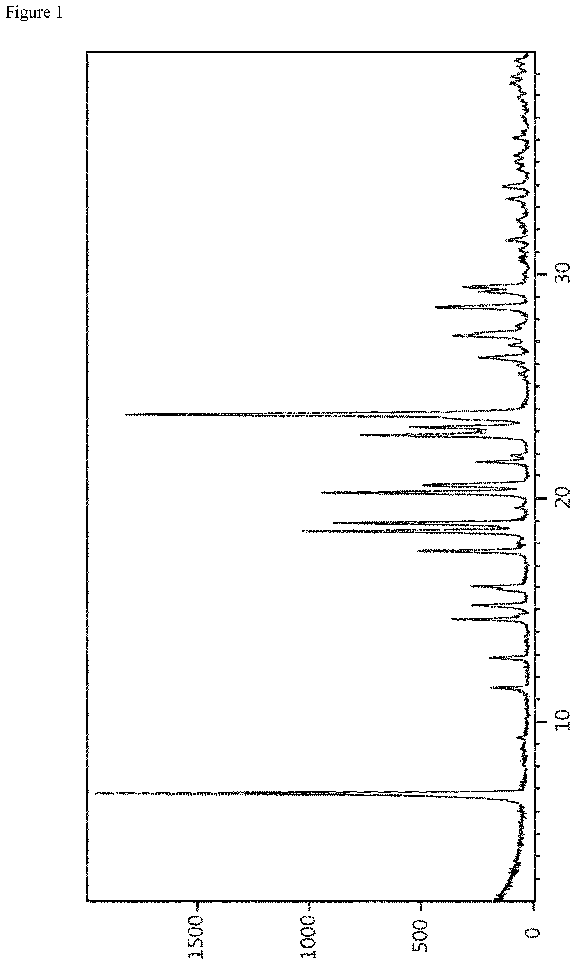

The inventors of the present invention surprisingly identified a polymorph of cabotegravir sodium, which is thermodynamically stable at room temperature. This form is hereinafter referred to as "Form B". Aspects, advantageous features and preferred embodiments of the present invention are summarized in the following items:
1) A crystalline form of cabotegravir sodium characterized by having a powder X-ray diffractogram comprising reflections at 2-Theta angles of (6.8 ± 0.2)°, (18.5 ± 0.2)° and (23.7 ± 0.2)°, when measured at a temperature in the range of from 20 to 30 °C with Cu-Kalpha_{1,2} radiation having a wavelength of 0.15419 nm.
2) A crystalline form of cabotegravir sodium characterized by having a powder X-ray diffractogram comprising reflections at 2-Theta angles of (6.8 ± 0.2)°, (18.5 ± 0.2)°, (20.3 ± 0.2)° and (23.7 ± 0.2)°, when measured at a temperature in the range of from 20 to 30 °C with Cu-Kalpha_{1,2} radiation having a wavelength of 0.15419 nm.
3) A crystalline form of cabotegravir sodium characterized by having a powder X-ray diffractogram comprising reflections at 2-Theta angles of (6.8 ± 0.2)°, (18.5 ± 0.2)°, (18.9 ± 0.2)°, (20.3 ± 0.2)° and (23.7 ± 0.2)°, when measured at a temperature in the range of from 20 to 30 °C with Cu-Kalpha_{1,2} radiation having a wavelength of 0.15419 nm.
4) A crystalline form of cabotegravir sodium characterized by having a powder X-ray diffractogram comprising reflections at 2-Theta angles of (6.8 ± 0.2)°, (18.5 ± 0.2)°, (18.9 ± 0.2)°, (20.3 ± 0.2)°, (22.8 ± 0.2)° and (23.7 ± 0.2)°, when measured at a temperature in the range of from 20 to 30 °C with Cu-Kalpha_{1,2} radiation having a wavelength of 0.15419 nm.
5) A crystalline form of cabotegravir sodium characterized by having a powder X-ray diffractogram comprising reflections at 2-Theta angles (6.8 ± 0.2)°, (18.5 ± 0.2)°, (18.9 ± 0.2)°, (20.3 ± 0.2)°, (22.8 ± 0.2)°, (23.2 ± 0.2)° and (23.7 ± 0.2)°, when measured at a temperature in the range of from 20 to 30 °C with Cu-Kalpha_{1,2} radiation having a wavelength of 0.15419 nm.
6) A crystalline form of cabotegravir sodium characterized by having a powder X-ray diffractogram comprising reflections at 2-Theta angles of (6.8 ± 0.2)°, (17.7 ± 0.2)°, (18.5 ± 0.2)°, (18.9 ± 0.2)°, (20.3 ± 0.2)°, (22.8 ± 0.2)°, (23.2 ± 0.2)° and (23.7 ± 0.2)°, when measured at a temperature in the range of from 20 to 30 °C with Cu-Kalpha_{1,2} radiation having a wavelength of 0.15419 nm.
7) A crystalline form of cabotegravir sodium characterized by having a powder X-ray diffractogram comprising reflections at 2-Theta angles of (6.8 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)° and (23.7 ± 0.2)°, when measured at a temperature in the range of from 20 to 30 °C with Cu-Kalpha_{1,2} radiation having a wavelength of 0.15419 nm.
8) A crystalline form of cabotegravir sodium characterized by having a powder X-ray diffractogram comprising reflections at 2-Theta angles of (6.8 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)°, (23.7 ± 0.2)° and (28.6 ± 0.2)°, when measured at a temperature in the range of from 20 to 30 °C with Cu-Kalpha_{1,2} radiation having a wavelength of 0.15419 nm.
9) A crystalline form of cabotegravir sodium characterized by having a powder X-ray diffractogram comprising reflections at 2-Theta angles of (6.8 ± 0.2)°, (14.6 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)°, (23.7 ± 0.2)° and (28.6 ± 0.2)°, when measured at a temperature in the range of from 20 to 30 °C with Cu-Kalpha_{1,2} radiation having a wavelength of 0.15419 nm.
10) A crystalline form of cabotegravir sodium characterized by having a powder X-ray diffractogram comprising reflections at 2-Theta angles of (6.8 ± 0.2)°, (14.6 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)°, (23.7 ± 0.2)°, (27.3 ± 0.2)° and (28.6 ± 0.2)°, when measured at a temperature in the range of from 20 to 30 °C with Cu-Kalpha_{1,2} radiation having a wavelength of 0.15419 nm.
11) A crystalline form of cabotegravir sodium characterized by having a powder X-ray diffractogram comprising reflections at 2-Theta angles of (6.8 ± 0.1)°, (18.5 ± 0.1)° and (23.7 ± 0.1)°, when measured at a temperature in the range of from 20 to 30 °C with Cu-Kalpha_{1,2} radiation having a wavelength of 0.15419 nm.
12) A crystalline form of cabotegravir sodium characterized by having a powder X-ray diffractogram comprising reflections at 2-Theta angles of (6.8 ± 0.1)°, (18.5 ± 0.1)°, (20.3 ± 0.1)° and (23.7 ± 0.1)°, when measured at a temperature in the range of from 20 to 30 °C with Cu-Kalpha_{1,2} radiation having a wavelength of 0.15419 nm.
13) A crystalline form of cabotegravir sodium characterized by having a powder X-ray diffractogram comprising reflections at 2-Theta angles of (6.8 ± 0.1)°, (18.5 ± 0.1)°, (18.9 ± 0.1)°, (20.3 ± 0.1)° and (23.7 ± 0.1)°, when measured at a temperature in the range of from 20 to 30 °C with Cu-Kalpha_{1,2} radiation having a wavelength of 0.15419 nm.
14) A crystalline form of cabotegravir sodium characterized by having a powder X-ray diffractogram comprising reflections at 2-Theta angles of (6.8 ± 0.1)°, (18.5 ± 0.1)°, (18.9 ± 0.1)°, (20.3 ± 0.1)°, (22.8 ± 0.1)° and (23.7 ± 0.1)°, when measured at a temperature in the range of from 20 to 30 °C with Cu-Kalpha_{1,2} radiation having a wavelength of 0.15419 nm.
15) A crystalline form of cabotegravir sodium characterized by having a powder X-ray diffractogram comprising reflections at 2-Theta angles (6.8 ± 0.1)°, (18.5 ± 0.1)°, (18.9 ± 0.1)°, (20.3 ± 0.1)°, (22.8 ± 0.1)°, (23.2 ± 0.1)° and (23.7 ± 0.1)°, when measured at a temperature in the range of from 20 to 30 °C with Cu-Kalpha_{1,2} radiation having a wavelength of 0.15419 nm.
16) A crystalline form of cabotegravir sodium characterized by having a powder X-ray diffractogram comprising reflections at 2-Theta angles of (6.8 ± 0.1)°, (17.7 ± 0.1)°, (18.5 ± 0.1)°, (18.9 ± 0.1)°, (20.3 ± 0.1)°, (22.8 ± 0.1)°, (23.2 ± 0.1)° and (23.7 ± 0.1)°, when measured at a temperature in the range of from 20 to 30 °C with Cu-Kalpha_{1,2} radiation having a wavelength of 0.15419 nm.
17) A crystalline form of cabotegravir sodium characterized by having a powder X-ray diffractogram comprising reflections at 2-Theta angles of (6.8 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)° and (23.7 ± 0.1)°, when measured at a temperature in the range of from 20 to 30 °C with Cu-Kalpha_{1,2} radiation having a wavelength of 0.15419 nm.
18) A crystalline form of cabotegravir sodium characterized by having a powder X-ray diffractogram comprising reflections at 2-Theta angles of (6.8 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)°, (23.7 ± 0.1)° and (28.6 ± 0.1)°, when measured at a temperature in the range of from 20 to 30 °C with Cu-Kalpha_{1,2} radiation having a wavelength of 0.15419 nm.
19) A crystalline form of cabotegravir sodium characterized by having a powder X-ray diffractogram comprising reflections at 2-Theta angles of (6.8 ± 0.1)°, (14.6 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)°, (23.7 ± 0.1)° and (28.6 ± 0.1)°, when measured at a temperature in the range of from 20 to 30 °C with Cu-Kalpha_{1,2} radiation having a wavelength of 0.15419 nm.
20) A crystalline form of cabotegravir sodium characterized by having a powder X-ray diffractogram comprising reflections at 2-Theta angles of (6.8 ± 0.1)°, (14.6 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)°, (23.7 ± 0.1)°, (27.3 ± 0.1)° and (28.6 ± 0.1)°, when measured at a temperature in the range of from 20 to 30 °C with Cu-Kalpha_{1,2} radiation having a wavelength of 0.15419 nm.
21) A crystalline form of cabotegravir sodium characterized by having a powder X-ray diffractogram essentially the same as shown in Figure 1 of the present invention, when measured at a temperature in the range of from 20 to 30 °C with Cu-Kalpha_{1,2} radiation having a wavelength of 0.15419 nm.
22) A composition comprising the crystalline form of cabotegravir sodium according to any one of the preceding items, which composition is essentially free of any other physical form of cabotegravir sodium.
23) A composition comprising the crystalline form of cabotegravir sodium according to any one of items 1 to 21, characterized by comprising at most 20 weight% of any other physical form of cabotegravir sodium, based on the weight of the composition.
24) A composition comprising the crystalline form of cabotegravir sodium according to any one of items 1 to 21, characterized by comprising at most 10 weight% of any other physical form of cabotegravir sodium, based on the weight of the composition.
25) A composition comprising the crystalline form of cabotegravir sodium according to any one of items 1 to 21, characterized by comprising at most 5 weight% of any other physical form of cabotegravir sodium, based on the weight of the composition.
26) A composition comprising the crystalline form of cabotegravir sodium according to any one of items 1 to 21, characterized by comprising at most 2 weight% of any other physical form of cabotegravir sodium, based on the weight of the composition.
27) A composition comprising the crystalline form of cabotegravir sodium according to any one of items 1 to 21, characterized by comprising at most 1 weight% of any other physical form of cabotegravir sodium, based on the weight of the composition.
28) The composition according to any one of items 22 to 27, wherein the any other physical form of cabotegravir sodium is Form A characterized by having a powder X-ray diffractogram comprising reflections at 2-Theta angles of (5.4 ± 0.2)°, (12.8 ± 0.2)°, (13.1 ± 0.2)°, (23.9 ± 0.2)° and (24.4 ± 0.2)°, when measured at a temperature in the range of from 20 to 30 °C with Cu-Kalpha_{1,2} radiation having a wavelength of 0.15419 nm.
29) The composition according to any one of items 22 to 27, wherein the any other physical form of cabotegravir sodium is amorphous.
30) A process for the preparation of the crystalline form of cabotegravir sodium as defined in any one of items 1 to 21 or the composition as defined in any one of items 22 to 29 comprising:
   (i) providing a crystalline form of cabotegravir sodium (Form A) characterized by having a PXRD comprising reflections at 2-Theta angles of (5.4 ± 0.2)°, (12.8 ± 0.2)°, (13.1 ± 0.2)°, (23.9 ± 0.2)° and (24.4 ± 0.2)°, when measured at a temperature in the range of from 20 to 30 °C with Cu-Kalpha_{1,2} radiation having a wavelength of 0.15419 nm.
   (ii) slurrying the crystalline form of cabotegravir sodium provided in step (i) in a solvent selected from the group consisting of cyclic ethers, C₃-C₄ ketones and methyl acetate or mixtures thereof, wherein the slurrying is for a time sufficient to effect transformation of cabotegravir sodium (Form A) to the crystalline form of cabotegravir sodium as defined in any one of items 1 to 21 or the composition as defined in any one of items 22 to 29.
31) The process of item 30, wherein the cyclic ethers are selected from 1,4-dioxane and tetrahydrofuran.
32) The process of item 30, wherein the C₃-C₄ ketones are selected from acetone and 2-butanone.
33) The process according to any one of items 30 to 32, wherein cabotegravir sodium concentration of the suspension provided in step (ii) is in the range of from 5 to 80g/L.
34) The process according to any one of items 30 to 32, wherein the cabotegravir sodium concentration of the suspension provided in step (ii) is in the range of from 5 to 50 g/L.
35) The process according to any one of items 30 to 32, wherein the cabotegravir sodium concentration of the suspension provided in step (ii) is in the range of from 5 to 25 g/L.
36) The process according to any one of items 30 to 32, wherein cabotegravir sodium concentration of the suspension provided in step (ii) is 10 g/L.
37) The process according to any one of items 30 to 36, wherein slurrying is performed at a temperature in the range of from 20 to 30 °C.
38) The process according to any one of items 30 to 36, wherein slurrying is performed at a temperature in the range of from 40 to 80 °C.
39) The process according to any one of items 30 to 36, wherein slurrying is performed at a temperature in the range of from 40 to 60 °C.
40) The process according to any one of items 30 to 39, wherein the slurrying is performed for at least 24 hours.
41) The process according to item 40, wherein the slurrying is performed for at least 120 hours.
42) The process according to any one of items 30 to 41, further comprising step (iii) separating at least a part of the crystals obtained in step (ii) from the mother liquor.
43) The process according to item 42, wherein the crystals are separated from the mother liquor by filtration, centrifugation, decantation or solvent evaporation.
44) The process according to item 42, wherein the crystals are separated from the mother liquor by filtration or centrifugation.
45) The process according to item 42, wherein the crystals are separated from the mother liquor by filtration.
46) The process according to any one of items 42 to 45, further comprising step (iv) washing the isolated crystals obtained in step (iii).
47) The process according to item 46, wherein the crystals are washed with an organic solvent and/or water.
48) The process according to item 47, wherein the organic solvent is selected from the group consisting of 1,4-dioxane, tetrahydrofuran, acetone, 2-butanone and methyl acetate or mixtures thereof.
49) The process according to any one of items 30 to 48, further comprising step (v) drying the crystals obtained in any one of steps (ii) to (iv).
50) The process according to item 49, wherein drying is performed at a temperature of 150 °C or less.
51) The process according to item 49, wherein drying is performed at a temperature of 100 °C or less.
52) The process according to item 49, wherein drying is performed at a temperature of 60 °C or less.
53) The process according to item 49, wherein drying is performed at a temperature of 40 °C or less.
54) The process according to item 49, wherein drying is performed at a temperature in the range of from 20 to 30 °C.
55) The process according to any one of items 49 to 54, wherein drying is performed for a period in the range of from 1 to 72 hours.
56) The process according to any one of items 49 to 54, wherein drying is performed for a period in the range of from 2 to 48 hours.
57) The process according to any one of items 49 to 54, wherein drying is performed for a period in the range of from 4 to 24 hours.
58) The process according to any one of items 49 to 54, wherein drying is performed for a period in the range of from 6 to 18 hours.
59) Use of the crystalline form of cabotegravir sodium as defined in any one of items 1 to 21 for the preparation of a pharmaceutical composition.
60) Use of the crystalline form of cabotegravir sodium as obtained by the process according to any one of items 30 to 58 for the preparation of a pharmaceutical composition.
61) Use of the composition as defined in any one of items 22 to 29 for the preparation of a pharmaceutical composition.
62) Use of the composition as obtained by the process according to any one of items 30 to 58 for the preparation of a pharmaceutical composition.
63) The use according to any one of items 59 to 62, wherein the pharmaceutical composition is prepared by a wet or dry processing method.
64) The use according to item 63, wherein the wet processing method comprises wet granulation.
65) The use according to item 63, wherein the dry processing method comprises dry granulation or dry compaction.
66) A pharmaceutical composition comprising the crystalline form of cabotegravir sodium as defined in any one of items 1 to 21 or the composition as defined in any one of items 22 to 29 and at least one pharmaceutically acceptable excipient.
67) The pharmaceutical composition of item 66, comprising a predetermined and/or effective amount of the crystalline form of cabotegravir sodium as defined in any one of items 1 to 21 or the composition as defined in any one of items 22 to 29 and at least one pharmaceutically acceptable excipient.
68) The pharmaceutical composition of item 66 or 67, wherein the at least one pharmaceutically acceptable excipient is selected from the group consisting of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof.
69) The pharmaceutical composition according to any one of items 66 to 68, wherein the at least one pharmaceutically acceptable excipient is selected from the group consisting of mannitol, microcrystalline cellulose, povidone, sodium starch glycolate and sodium stearyl fumarate.
70) The pharmaceutical composition according to any one of items 66 to 69 comprising one or more additional active pharmaceutical ingredient(s).
71) The pharmaceutical composition according to item 70, wherein the one or more additional pharmaceutical active ingredient(s) is/ are selected from the group consisting of entry/fusion inhibitors, reverse transcriptase inhibitors (RTIs), integrase strand transfer inhibitors (INSTIs), maturation inhibitors, protease inhibitors (PIs) or any combinations thereof.
72) The pharmaceutical composition according to item 71, wherein the entry/fusion inhibitors are selected from the group consisting of enfuvirtide, maraviroc, vicriviroc, cenicriviroc, ibalizumab and fostemsavir or mixtures thereof.
73) The pharmaceutical composition according to item 71, wherein the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof.
74) The pharmaceutical composition according to item 71, wherein the integrase strand transfer inhibitors (INSTIs) are selected from the group consisting of dolutegravir, elvitegravir, raltegravir and bictegravir or mixtures thereof.
75) The pharmaceutical composition according to item 71, wherein the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.
76) The pharmaceutical composition according to item 70, wherein the one or more additional active pharmaceutical ingredient is rilpivirine.
77) The pharmaceutical composition according to item 70, wherein the one or more additional active pharmaceutical ingredient is rilpivirine in form of its hydrochloride salt.
78) The pharmaceutical composition according to item 70, wherein the one or more additional active pharmaceutical ingredient is abacavir and/or lamivudine.
79) The pharmaceutical composition according to any one of items 66 to 78 which is an oral solid dosage form.
80) The pharmaceutical composition according to item 79, wherein the oral solid dosage form is a tablet or a capsule.
81) The pharmaceutical composition according to any one of items 66 to 80, wherein the crystalline form of cabotegravir sodium as defined in any one of items 1 to 21 is present in an amount selected from the group consisting of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg and 90 mg, calculated as cabotegravir.
82) The pharmaceutical composition according to item 81, wherein the crystalline form of cabotegravir sodium as defined in any one of items 1 to 21 is present in an amount of 30 mg, calculated as cabotegravir.
83) The pharmaceutical composition according to any one of items 66 to 82, which is administered once daily.
84) The pharmaceutical composition according to any one of items 66 to 83 for use as a medicament.
85) The pharmaceutical composition according to any one of items 66 to 83 for use in the treatment and/or prophylaxis of viral infections.
86) The pharmaceutical composition according to item 85, wherein the viral infection is caused by DNA viruses, RNA viruses, herpesviruses, retroviruses, hepadnaviruses, papillomavirus, hantavirus, adenoviruses and HIV.
87) The crystalline form of cabotegravir sodium as defined in any one of items 1 to 21 characterized by having a powder X-ray diffractogram not comprising a reflection at a 2-Theta angle of (5.4 ± 0.2)° 2-Theta.

### Definitions

The term "cabotegravir" as used herein refers to (3*S*,11a*R*)-*N*-[2,4-Difluorophenyl)methyl]-6-hydroxy-3-methyl-5,7-dioxo-2,3,5, 7,11,11a-hexahydro[1,3]oxazolo[3,2-*a*]pyrido[1,2-*d*]pyrazine-8-carboxamide according to Formula (I) disclosed herein above.

The term "cabotegravir sodium" as used herein refers to the sodium salt of cabotegravir, having a chemical structure, wherein about one mol of cabotegravir is associated with one mol of sodium via ionic interaction of deprotonated cabotegravir with Na⁺. Cabotegravir sodium can be represented by the chemical structure according to Formula (II) herein after.

The term "cabotegravir sodium Form A" as used herein, refers to the crystalline form of cabotegravir sodium, which is for example inherently disclosed in Example Z-9 of WO 2006/116764 A1, Example Ae of WO 2010/068253 A1 and Example 17 of WO 2015/177537 A1. Form A of cabotegravir sodium can be characterized by having a powder X-ray diffractogram comprising reflections at 2-Theta angles of (5.4 ± 0.2)°, (12.8 ± 0.2)°, (13.1 ± 0.2)°, (23.9 ± 0.2)° and (24.4 ± 0.2)°, when measured at a temperature in the range of from 20 to 30 °C with Cu-Kalpha_{1,2} radiation having a wavelength of 0.15419 nm.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-80% relative humidity, preferably 30-70% relative humidity, more preferably 40-60% relative humidity and most preferably 50% relative humidity.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

As used herein, the term "amorphous" refers to a solid form of a compound that is not crystalline. An amorphous compound possesses no long-range order and does not display a definitive X-ray diffraction pattern with reflections.

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-theta. Thus, a reflection that usually appears at 6.8° 2-Theta for example can appear between 6.6° and 7.0° 2-theta, preferably between 6.7 and 6.9° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

As used herein, the term "substantially free of any other physical form" with reference to a composition comprising a particular physical form of cabotegravir sodium means that the composition includes at most 20%, preferably at most 10%, more preferably at most 5%, even more preferably at most 2% and most preferably at most 1% by weight of any other physical form of cabotegravir sodium, based on the weight of the composition.

The term "physical form" as used herein refers to any crystalline and/or amorphous phase of a compound.

Crystalline forms of cabotegravir sodium may be referred to herein as being characterized by a powder X-ray diffractogram "as shown in" a figure. The person skilled in the art understands that factors such as variations in instrument type, response and variations in sample directionality, sample concentration, sample purity, sample history and sample preparation may lead to variations, for example relating to the exact reflection positions and intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid from said solution.

A "predetermined amount" as used herein with regard to cabotegravir sodium refers to the initial amount of cabotegravir sodium used for the preparation of a pharmaceutical composition having a desired dosage strength of cabotegravir.

The term "effective amount" as used herein with regard to cabotegravir sodium encompasses an amount of cabotegravir sodium, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

### BRIEF DESCRIPTION OF THE FIGURES

**Figure 1****:** illustrates a representative powder X-ray diffractogram of cabotegravir sodium Form B of the present invention. The x-axis shows the scattering angle in °2-Theta, the y-axis shows the intensity of the scattered X-ray beam in counts of detected photons.
**Figure 2****:** illustrates a representative powder X-ray diffractogram of cabotegravir sodium Form A prepared according to Reference Example 1 herein. The x-axis shows the scattering angle in °2-Theta, the y-axis shows the intensity of the scattered X-ray beam in counts of detected photons.
**Figure 3****:** illustrates a comparison of a representative powder X-ray diffractogram of crystalline Form B of cabotegravir sodium of the present invention (bottom) and a representative PXRD of Form A of cabotegravir sodium prepared according to Reference Example 1 herein (top). The x-axis shows the scattering angle in °2-Theta. The powder X-ray diffractogram of Form A was shifted along the y-axis to separate the diffractograms for clarity. The y-axis is therefore arbitrary and was not labeled.

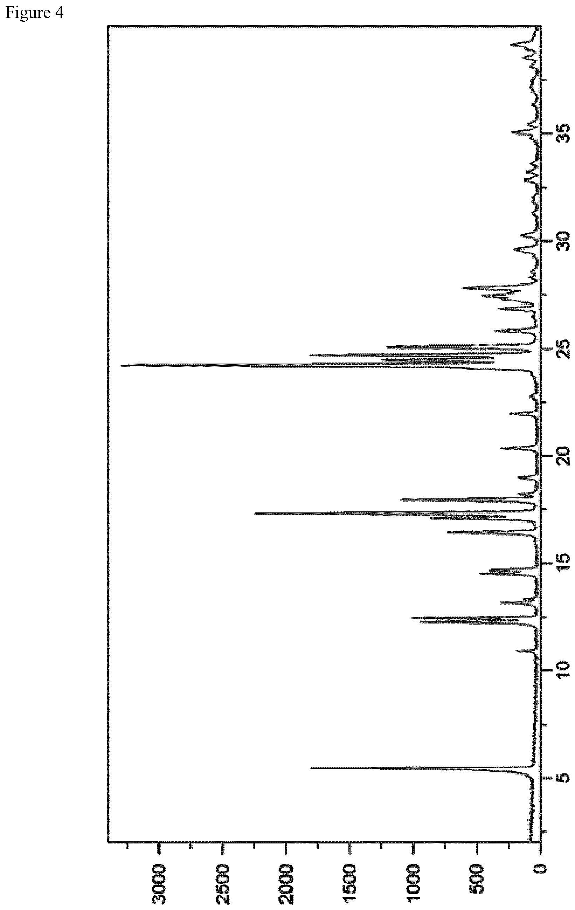

**Figure 4****:** illustrates a representative powder X-ray diffractogram of crystalline cabotegravir prepared according to Example D of WO 2011/119566 A1. The x-axis shows the scattering angle in °2-Theta, the y-axis shows the intensity of the scattered X-ray beam in counts of detected photons.

### DETAILED DESCRIPTION OF THE INVENTION

The present invention provides a novel crystalline form of cabotegravir sodium, herein also referred to as "Form B".

The present inventors have surprisingly found that Form B of the present invention is thermodynamically more stable than prior art Form A, at least at room temperature in the presence of particular organic solvents. The provision of a thermodynamically stable modification of cabotegravir sodium is highly desirable due to the fact that the risk of phase transitions, which may occur during manufacture and/or storage of a drug product, such as polymorphic conversions and/or amorphization, is minimized. Such transitions can in general have serious consequences for the pharmaceutical product with regard to safety and efficacy. Hence, Form B of the present invention is a favored solid form of cabotegravir sodium, to be used for the preparation of a pharmaceutical drug product. This is because the usage of Form B ensures a reliable safety and efficacy profile of a drug product containing Form B during the whole shelf-life of the product.

Cabotegravir sodium can be represented by the following chemical structure according to Formula (II)

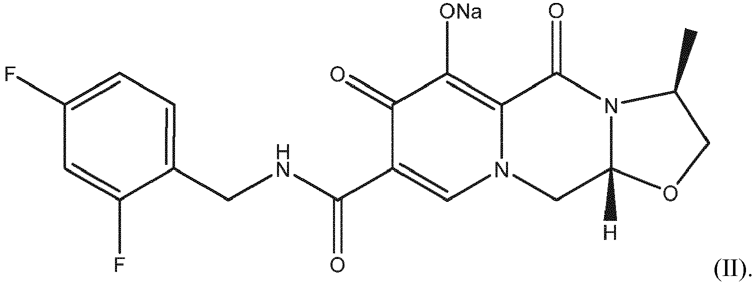

Cabotegravir sodium can be characterized by a molar ratio of cabotegravir and sodium preferably in the range of from 1.0 : 0.7 to 1.0 : 1.3, more preferably in the range of from 1.0 : 0.8 to 1.0 : 1.2, even more preferably in the range of from 1.0 : 0.9 to 1.0 : 1.1 and in particular the molar ratio is 1.0 : 1.0.

Cabotegravir sodium Form B of the present invention may be characterized by any one of the following embodiments or by combining two or more of the following embodiments.

Hence, in a first aspect the present invention relates to a crystalline form of cabotegravir sodium (Form B) which can be characterized by having a powder X-ray diffractogram comprising reflections at 2-Theta angles of:
(6.8 ± 0.2)°, (18.5 ± 0.2)° and (23.7 ± 0.2)°; or
(6.8 ± 0.2)°, (18.5 ± 0.2)°, (20.3 ± 0.2)° and (23.7 ± 0.2)°; or
(6.8 ± 0.2)°, (18.5 ± 0.2)°, (18.9 ± 0.2)°, (20.3 ± 0.2)° and (23.7 ± 0.2)°; or
(6.8 ± 0.2)°, (18.5 ± 0.2)°, (18.9 ± 0.2)°, (20.3 ± 0.2)°, (22.8 ± 0.2)° and (23.7 ± 0.2)°; or
(6.8 ± 0.2)°, (18.5 ± 0.2)°, (18.9 ± 0.2)°, (20.3 ± 0.2)°, (22.8 ± 0.2)°, (23.2 ± 0.2)° and (23.7 ± 0.2)°; or
(6.8 ± 0.2)°, (17.7 ± 0.2)°, (18.5 ± 0.2)°, (18.9 ± 0.2)°, (20.3 ± 0.2)°, (22.8 ± 0.2)°, (23.2 ± 0.2)° and (23.7 ± 0.2)°; or
(6.8 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)° and (23.7 ± 0.2)°; or
(6.8 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)°, (23.7 ± 0.2)° and (28.6 ± 0.2)°; or
(6.8 ± 0.2)°, (14.6 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)°, (23.7 ± 0.2)° and (28.6 ± 0.2)°; or
(6.8 ± 0.2)°, (14.6 ± 0.2)°, (17.7 ± 0.2)°, (18.5 ± 0.2)°, (18.9 ± 0.2)°, (20.3 ± 0.2)°, (20.6 ± 0.2)°, (22.8 ± 0.2)°, (23.2 ± 0.2)°, (23.7 ± 0.2)°, (27.3 ± 0.2)° and (28.6 ± 0.2)°;
when measured at a temperature in the range of from 20 to 30 °C with Cu-Kalpha_{1,2} radiation having a wavelength of 0.15419 nm.

In another embodiment, the present invention relates to a crystalline form of cabotegravir sodium (Form B) which can be characterized by having a powder X-ray diffractogram comprising reflections at 2-Theta angles of:
(6.8 ± 0.1)°, (18.5 ± 0.1)° and (23.7 ± 0.1)°; or
(6.8 ± 0.1)°, (18.5 ± 0.1)°, (20.3 ± 0.1)° and (23.7 ± 0.1)°; or
(6.8 ± 0.1)°, (18.5 ± 0.1)°, (18.9 ± 0.1)°, (20.3 ± 0.1)° and (23.7 ± 0.1)°; or
(6.8 ± 0.1)°, (18.5 ± 0.1)°, (18.9 ± 0.1)°, (20.3 ± 0.1)°, (22.8 ± 0.1)° and (23.7 ± 0.1)°; or
(6.8 ± 0.1)°, (18.5 ± 0.1)°, (18.9 ± 0.1)°, (20.3 ± 0.1)°, (22.8 ± 0.1)°, (23.2 ± 0.1)° and (23.7 ± 0.1)°; or
(6.8 ± 0.1)°, (17.7 ± 0.1)°, (18.5 ± 0.1)°, (18.9 ± 0.1)°, (20.3 ± 0.1)°, (22.8 ± 0.1)°, (23.2 ± 0.1)° and (23.7 ± 0.1)°; or
(6.8 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)° and (23.7 ± 0.1)°; or
(6.8 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)°, (23.7 ± 0.1)° and (28.6 ± 0.1)°; or
(6.8 ± 0.1)°, (14.6 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)°, (23.7 ± 0.1)° and (28.6 ± 0.1)°; or
(6.8 ± 0.1)°, (14.6 ± 0.1)°, (17.7 ± 0.1)°, (18.5 ± 0.1)°, (18.9 ± 0.1)°, (20.3 ± 0.1)°, (20.6 ± 0.1)°, (22.8 ± 0.1)°, (23.2 ± 0.1)°, (23.7 ± 0.1)°, (27.3 ± 0.1)° and (28.6 ± 0.1)°;
when measured at a temperature in the range of from 20 to 30 °C with Cu-Kalpha_{1,2} radiation having a wavelength of 0.15419 nm.

In yet another embodiment, the present invention relates to a crystalline form of cabotegravir sodium (Form B) characterized by having a powder X-ray diffractogram essentially the same as shown in Figure 1 of the present invention, when measured at a temperature in the range of from 20 to 30 °C with Cu-Kalpha_{1,2} radiation having a wavelength of 0.15419 nm.

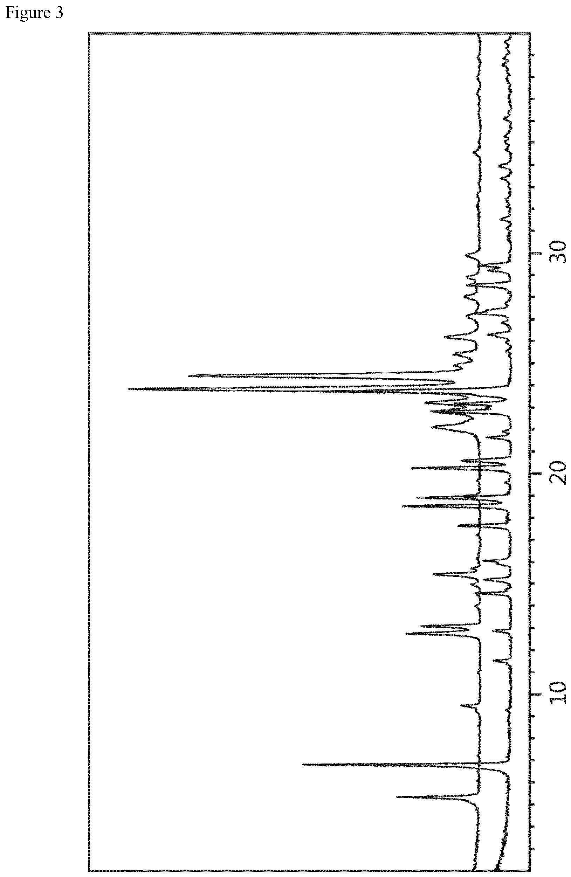

The powder X-ray diffractogram of cabotegravir sodium Form B of the present invention can be clearly distinguished from the one of prior art Form A (see also the overlay displayed in Figure 3 herein). Form B shows for example a reflection at (6.8 ± 0.2)° 2-Theta, whereas Form A shows no reflection in the same range. On the other hand, Form B shows no reflection at (5.4 ± 0.2)° 2-Theta, whereas Form A possesses a characteristic reflection in this range. Thus, in a another aspect, the present invention relates to a crystalline form of cabotegravir sodium (Form B) which can be characterized by having a powder X-ray diffractogram as described above, but not comprising a reflection at a 2-Theta angle of (5.4 ± 0.2)° 2-Theta.

In one aspect, the present invention relates to a composition comprising Form B of cabotegravir sodium of the present invention, which is essentially free of any other physical forms of cabotegravir sodium. For example, a composition comprising Form B of cabotegravir sodium of the present invention comprises at most 20 weight%, preferably at most 10 weight%, more preferably at most 5 weight%, even more preferably at most 2 weight% and most preferably at most 1 weight% of any other physical form of cabotegravir sodium, based on the weight of the composition. Preferably, the any other physical form of cabotegravir sodium is Form A as defined herein or amorphous.

In another aspect, the present invention relates to a process for the preparation of crystalline Form B of cabotegravir sodium of the present invention or the composition comprising Form B of cabotegravir sodium as defined above comprising:
(i) providing a crystalline form of cabotegravir sodium (Form A) characterized by having a PXRD comprising reflections at 2-Theta angles of (5.4 ± 0.2)°, (12.8 ± 0.2)°, (13.1 ± 0.2)°, (23.9 ± 0.2)° and (24.4 ± 0.2)°, when measured at a temperature in the range of from 20 to 30 °C with Cu-Kalpha_{1,2} radiation having a wavelength of 0.15419 nm.
(ii) slurrying the crystalline form of cabotegravir sodium (Form A) provided in step (i) in a solvent selected from the group consisting of cyclic ethers, C₃-C₄ ketones and methyl acetate or mixtures thereof, wherein the slurrying is for a time sufficient to effect transformation of cabotegravir sodium (Form A) to crystalline Form B of cabotegravir sodium of the present invention;
(iii)optionally separating at least a part of the crystals obtained in step (ii) from their mother liquor;
(iv)optionally washing the isolated crystals obtained in step (iii);
(v) optionally drying the crystals obtained in any one of steps (ii) to (iv);

Cabotegravir sodium Form A, which is applied as starting material in the above described process can be prepared according to the procedures disclosed in Example Z-1 of WO 2006/116764 A1, Example Ae of WO 2010/068253 A, Example 17 of WO 2015/177537 A1 or according to the teaching of J. Med. Chem. 2013, 56, 5901-5916.

The solvent-mediated transformation of cabotegravir sodium Form A to cabotegravir sodium Form B may be accomplished by applying conditions as defined hereinafter.

A suitable solvent, which may be used in step (ii) of the above described process may be selected from the group consisting of cyclic ethers, C₃-C₄ ketones and methyl acetate or mixtures thereof. In a preferred embodiment, the cyclic ethers are selected from tetrahydrofuran and 1,4-dioxane and the C₃-C₄ ketones are selected from acetone and 2-butanone. The solvent or solvent mixture may comprise additional organic solvents and/or water. However, most preferably the only solvent present in the slurry is selected from the group consisting of tetrahydrofuran, 1,4-dioxane, acetone, 2-butanone and methyl acetate.

The cabotegravir sodium concentration of the suspension is preferably in the range of from about 5 to 80 g/L, more preferably from about 5 to 50 g/L and most preferably from about 5 to 25 g/L, for example the concentration is about 10 g/L.

Preferably, slurrying is performed at room temperature but depending on the applied concentration may also be conducted at elevated temperature for example at a temperature in the range of from about 40 to 80 °C, preferably from about 40 to 60 °C.

Slurrying encompasses any kind of movement of the solid material suspended in the solvent caused by, but not limited to e.g. agitation, stirring, mixing, shaking, vibration, sonication, wet milling and the like.

Slurrying is conducted for a time sufficient that at least a substantial part, preferably all of Form A has converted to Form B. Preferably slurrying is performed for a period in the range of several hours to several days. Slurrying may for example be performed for a period in the range of from 6 hours to 14 days or longer. The skilled person may monitor the conversion of cabotegravir sodium Form A to Form B by withdrawing samples from the slurry and analyzing the samples by powder X-ray diffraction.

Once cabotegravir sodium Form B is obtained or preferably obtained in essentially pure form, at least a part of the crystals is optionally separated from its mother liquor. Preferably, the crystals are separated from their mother liquor by any conventional method such as filtration, centrifugation, solvent evaporation or decantation, more preferably by filtration or centrifugation and most preferably by filtration.

Optionally, in a further step the isolated crystals are washed with a suitable solvent, for example an organic solvent and/or water. Suitable organic solvents comprise, but are not limited to tetrahydrofuran, 1,4-dioxane, acetone, 2-butanone and methyl acetate. Most preferably, the solvent applied in step (ii) of the above defined process is also used for washing.

The obtained crystals may then optionally be dried. Drying may be performed at a temperature of about 150 °C or less, preferably of about 100 °C or less, more preferably of about 60 °C or less and most preferably of about 40 °C or less. Typically, drying is performed at room temperature. Drying may be performed for a period in the range of from about 1 to 72 hours, preferably from 2 to 48 hours, more preferably from 4 to 24 hours and most preferably from 6 to 18 hours. Drying may be performed at ambient pressure and/ or under reduced pressure. Preferably, drying is performed at a pressure of about 100 mbar or less, more preferably of about 50 mbar or less and most preferably of about 30 mbar or less, for example a vacuum of about 20 mbar or less.

It was surprisingly found by the present inventors that prior art Form A of cabotegravir sodium undergoes a solvent-mediated phase transformation to a more stable modification, namely Form B of the present invention, when slurried for a sufficiently long time in the presence of a suitable organic solvent. This indicates that crystalline Form B of cabotegravir sodium is the thermodynamically more stable solid form at the tested conditions.

The thermodynamically most stable polymorphic form is preferably used for drug product development, because metastable polymorphs may therefore transform to the more stable form during pharmaceutical processing and/or upon storage. Such a phase change may cause formulation problems such as physical instability of the solid dosage form and changes in bioavailability. The usage of the thermodynamically most stable form is highly appreciated since the risk of polymorphic conversions can usually be minimized, and consistent quality and efficacy of the drug product can be provided. Hence, cabotegravir sodium Form B of the present invention is favored over Form A in order to ensure a safe and efficacious drug product for the patient.

Therefore, in a further aspect the present invention relates to the use of cabotegravir sodium Form B of the present invention as defined above for the preparation of a pharmaceutical composition.

The pharmaceutical composition of the present invention can be prepared by wet or dry processing methods. In certain embodiments the pharmaceutical composition is prepared by wet processing methods, such as, but not limited to, wet granulation methods. Suitable wet granulation methods comprise high-shear granulation or fluid-bed granulation. In another embodiment the pharmaceutical composition is prepared by dry processing methods, such as, but not limited to, direct compression or dry granulation methods. An example of dry granulation is roller compaction. The pharmaceutical composition obtained by dry or wet processing methods may be compressed into tablets, encapsulated or metered into sachets.

In a further aspect, the present invention relates to a pharmaceutical composition comprising cabotegravir sodium Form B as defined above, preferably in an effective and/or predetermined amount, and at least one pharmaceutically acceptable excipient and optionally one or more additional active pharmaceutical ingredient(s). Most preferably, the pharmaceutical composition of the present invention is an oral solid dosage form, such as a tablet or a capsule. Preferably, the pharmaceutical composition of the present invention is a tablet. In a preferred embodiment, the tablet is film-coated with a coating material containing polyvinyl alcohol (e.g. partially hydrolyzed), iron oxide (e.g. yellow), talc, and titanium dioxide.

The at least one pharmaceutically acceptable excipient, which is comprised in the pharmaceutical composition of the present invention, is preferably selected from the group consisting of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof.

In a preferred embodiment, the at least one pharmaceutically acceptable excipient is selected from the group consisting of mannitol, microcrystalline cellulose, povidone, sodium starch glycolate and sodium stearyl fumarate. In a preferred embodiment, all of these pharmaceutically acceptable excipients are comprised by the pharmaceutical composition of the present invention.

In another preferred embodiment, the one or more additional active pharmaceutical ingredient(s) is/are selected from the group consisting of entry/fusion inhibitors, reverse transcriptase inhibitors (RTIs), integrase strand transfer inhibitors (INSTI), maturation inhibitors, protease inhibitors (PIs) or mixtures thereof. In a further preferred embodiment, the entry/fusion inhibitors are selected from the group consisting of enfuvirtide, maraviroc, vicriviroc, cenicriviroc and fostemsavir or mixtures thereof, the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof, the integrase strand transfer inhibitors (INSTI) are selected from the group consisting of dolutegravir, elvitegravir, raltegravir and bictegravir or mixtures thereof, the maturation inhibitor is bevirimat and the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.

In a particular preferred embodiment, the one or more additional active pharmaceutical ingredient(s) is/are selected from the group consisting of rilpivirine and lamivudine, most preferably the one or more additional active pharmaceutical ingredient is rilpivirine e.g. in form of its hydrochloride salt.

Preferably, the present invention relates to a pharmaceutical composition as describe above, wherein the predetermined and/or effective amount of cabotegravir sodium is selected from the group consisting of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg and 90 mg calculated as cabotegravir. Most preferably, the invention relates to a pharmaceutical composition as describe above, wherein the predetermined and/or effective amount of cabotegravir sodium is 30 mg calculated as cabotegravir.

Preferably, the present invention relates to a pharmaceutical composition as described above, wherein the pharmaceutical composition is to be administered once-daily.

In a further aspect, the present invention relates to the pharmaceutical composition as described above for use as a medicament.

In yet another aspect, the present invention relates to the pharmaceutical composition as described above for use in the treatment or prophylaxis of viral infections caused by DNA viruses, RNA viruses, herpesviruses (e.g. CMV, HSV 1, HSV 2, VZV), retroviruses, hepadnaviruses (e.g. HBV), papillomavirus, hantavirus, adenoviruses and HIV.

In a particular embodiment, the present invention relates to the pharmaceutical composition as described above for use in the treatment or prophylaxis of HIV-1 infections.

In another aspect the present invention relates to the pharmaceutical composition as described above intended for the treatment of HIV-1 infections in combination with one or more additional active pharmaceutical ingredient(s) selected from the group consisting of entry/fusion inhibitors, reverse transcriptase inhibitors (RTIs), integrase strand transfer inhibitors (INSTI), maturation inhibitors, protease inhibitors (PIs) or mixtures thereof. In a further preferred embodiment, the entry/fusion inhibitors are selected from the group consisting of enfuvirtide, maraviroc, vicriviroc, cenicriviroc, ibalizumab and fostemsavir or mixtures thereof, the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof, the integrase strand transfer inhibitors (INSTI) are selected from the group consisting of dolutegravir, elvitegravir, raltegravir and bictegravir or mixtures thereof, the maturation inhibitor is bevirimat and the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.

A treatment in combination with one or more additional active pharmaceutical ingredient(s) can mean the administration of a pharmaceutical dosage form comprising the cabotegravir sodium form B of the present invention and the one or more additional active pharmaceutical ingredient(s) in the same dosage form, for example as a fixed-dose combination.

Alternatively, a treatment in combination with one or more additional active pharmaceutical ingredient(s) can mean the administration of separate pharmaceutical dosage forms, one comprising the cabotegravir sodium form B of the present invention, and the other(s) comprising the one or more additional active pharmaceutical ingredient(s) in separate dosage form(s). Typically in such a combination treatment instructions are provided that the pharmaceutical dosage form comprising the cabotegravir sodium form B of the present invention is to be administered in combination with said separate dosage form(s) for the effective treatment of a viral invention, such as HIV-1 infection.

### EXAMPLES

The following non-limiting examples are illustrative for the disclosure and are not to be construed as to be in any way limiting for the scope of the invention.

### Example 1: Preparation of cabotegravir sodium Form B

Cabotegravir sodium Form A (approximately 50 mg, prepared according to Reference Example 1 herein) was suspended in an organic solvent (5 mL) according to Table 1 and vigorously stirred at room temperature using a magnetic stirrer. After 7 days, the solids were collected by filtration dried under vacuum (30 mbar) at room temperature for 20 hours and analyzed by PXRD. The results are displayed in Table 1.

**Table 1: Overview equilibrium slurry experiments starting from cabotegravir sodium Form A**

| **Example** | **Solvent** | **Weighted sample** | **Result (PXRD)** |
|---|---|---|---|
| 1.1 | acetone | 46 mg Form A | Form B |
| 1.2 | 2-butanone | 47 mg Form A | Form B |
| 1.3 | methyl acetate | 47 mg Form A | Form B |
| 1.4 | tetrahydrofuran | 46 mg Form A | Form B |
| 1.5 | 1,4-dioxane | 54 mg Form A | Form B |

PXRD was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-theta at ambient conditions. A typical precision of the 2-theta values is in the range of ± 0.2° 2-Theta, preferably of ± 0.1° 2-Theta. Thus, the diffraction peak of cabotegravir sodium Form B that appears for example at 6.8° 2-Theta can appear in the range of from 6.6 to 7.0° 2-Theta, preferably in the range of from 6.7 to 6.9 ° 2-Theta on most X-ray diffractometers under standard conditions.

A representative diffractogram of cabotegravir sodium Form B is displayed in Figure 1 herein. The corresponding reflection list is provided in Table 2 below.

**Table 2: PXRD reflections and corresponding relative intensities of cabotegravir sodium Form B in the range of from 2 to 30° 2-Theta; A typical precision of the 2-Theta values is in the range of ± 0.2° 2-Theta, preferably of ± 0.1° 2-Theta.**

| **Reflection position [°2-Theta]** | **Relative intensity [%]** | **Reflection position [°2-Theta]** | **Relative intensity [%]** |
|---|---|---|---|
| 6.8 | 100 | 20.6 | 23 |
| 11.5 | 9 | 21.6 | 11 |
| 12.9 | 9 | 22.8 | 39 |
| 14.6 | 18 | 23.2 | 26 |
| 15.3 | 11 | 23.7 | 92 |
| 15.9 | 6 | 26.3 | 11 |
| 16.1 | 13 | 27.3 | 18 |
| 17.7 | 25 | 27.4 | 11 |
| 18.5 | 54 | 28.6 | 22 |
| 18.9 | 46 | 29.2 | 12 |
| 20.3 | 49 | 29.4 | 13 |

### Reference Example 1: Experimental repetition of the final step of Example Z-9

Cabotegravir (278 mg, 0.66 mmol e.g. prepared according to the procedure disclosed in Example Z-9, which is referring to Example Z-1 of WO 2006/116764 A1) was taken up in ethanol (10 mL) and treated with 1 N sodium hydroxide (aq) (0.66 mL, 0.66 mmol). The resulting suspension was stirred at room temperature for 30 minutes. Ether was added and the solid was collected by filtration. The obtained white solid was investigated by PXRD and found to be crystalline. Said crystalline material is designated Form A in the course of the present invention.

PXRD was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-theta at ambient conditions.

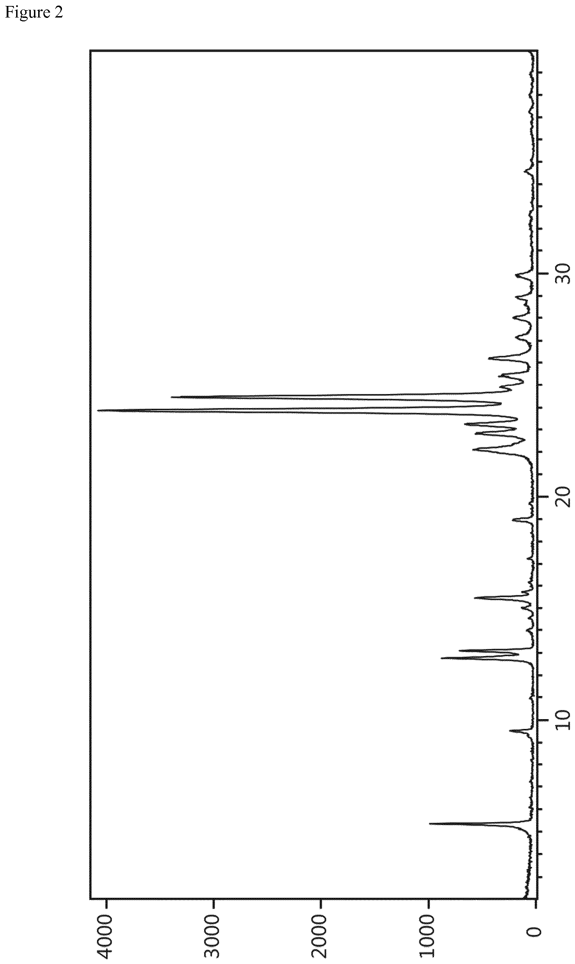

A representative diffractogram of cabotegravir sodium Form A is displayed in Figure 2 herein. The corresponding reflection list is provided in Table 3 below.

**Table 3: PXRD reflections and corresponding relative intensities of cabotegravir sodium Form A in the range of from 2 to 30° 2-Theta; A typical precision of the 2-Theta values is in the range of ± 0.2° 2-Theta, preferably of ± 0.1° 2-Theta.**

| **Reflection position [°2-Theta]** | **Relative intensity [%]** | **Reflection position [°2-Theta]** | **Relative intensity [%]** |
|---|---|---|---|
| 5.4 | 25 | 22.8 | 13 |
| 9.5 | 6 | 23.3 | 16 |
| 12.8 | 21 | 23.9 | 100 |
| 13.1 | 18 | 24.4 | 81 |
| 15.5 | 13 | 24.9 | 8 |
| 19.0 | 5 | 25.5 | 7 |
| 22.1 | 13 | 26.2 | 10 |

### Reference Example 2: Experimental repetition of Example Ae of WO 2010/068253 A1

After dissolution of cabotegravir (254 mg, 0.60 mmol, e.g. prepared according to Example Aa to Ad of WO 2010/068253 A1) in ethanol (40 mL) and water (10 mL) by heating, followed by filtration, 1 N sodium hydroxide (aq) (0.61 mL, 0.61 mmol) was added to the solution at 75 °C. The solution was gradually cooled to room temperature. Filtration, washing with ethanol (1.25 mL) and drying provided dolutegravir sodium crystals. The obtained crystals were analyzed by PXRD and could be assigned to Form A.

### Reference Example 3: Experimental repetition of J. Med. Chem. 2013, 56, 5901-5916

Cabotegravir (385 mg, 0.95 mmol) was treated with 1.0 N sodium hydroxide (aq) (0.95 mL, 0.95 mmol) in ethanol (15 mL) to obtain cabotegravir sodium (380 mg, yield 93% of theory) as a white solid. The obtained material was analyzed by PXRD and could be assigned to Form A.

### Reference Example 4: Salt formation in methanol

Cabotegravir (1002 mg, 2.47 mmol) was treated with 2.0 *N* sodium hydroxide (aq) (1.25 mL, 2.50 mmol) in methanol (60 mL) at 60 °C and the reaction mixture was stirred further for 1 hour. The reaction mixture was cooled to room temperature and stirred for 1 hour. The solid was isolated by filtration, washed with methanol and dried. The obtained material was analyzed by PXRD and could be assigned to Form A.

## Claims

1. A crystalline form of cabotegravir sodium **characterized by** having a powder X-ray diffractogram comprising reflections at 2-Theta angles of: (6.8 ± 0.2)°, (18.5 ± 0.2)° and (23.7 ± 0.2)°, when measured at a temperature in the range of from 20 to 30 °C with Cu-Kalpha_{1,2} radiation having a wavelength of 0.15419 nm.

2. The crystalline form of claim 1, further **characterized**
(i) **by** having a powder X-ray diffractogram comprising reflections at 2-Theta angles of: (6.8 ± 0.2)°, (18.5 ± 0.2)°, (20.3 ± 0.2)° and (23.7 ± 0.2)°, when measured at a temperature in the range of from 20 to 30 °C with Cu-Kalpha_{1,2} radiation having a wavelength of 0.15419 nm; or
(ii) by having a powder X-ray diffractogram comprising reflections at 2-Theta angles of: (6.8 ± 0.2)°, (18.5 ± 0.2)°, (18.9 ± 0.2)°, (20.3 ± 0.2)° and (23.7 ± 0.2)°, when measured at a temperature in the range of from 20 to 30 °C with Cu-Kalpha_{1,2} radiation having a wavelength of 0.15419 nm.

3. The crystalline form of claim 1 or claim 2, **characterized by** having a powder X-ray diffractogram the same as shown in Figure 1 of the present invention, when measured at a temperature in the range of from 20 to 30 °C with Cu-Kalpha_{1,2} radiation having a wavelength of 0.15419 nm.

4. A composition comprising the crystalline form according to any one of claims 1 to 3 and at most 20 weight% of any other physical form of cabotegravir sodium, based on the weight of the composition, preferably comprising at most 10 weight%, 5 weight%, 2 weight% or 1 weight% of any other physical form of cabotegravir sodium, based on the weight of the composition.

5. The composition according to claim 4, wherein the other physical form of cabotegravir sodium is Form A **characterized by** having a powder X-ray diffractogram comprising reflections at 2-Theta angles of (5.4 ± 0.2)°, (12.8 ± 0.2)°, (13.1 ± 0.2)°, (23.9 ± 0.2)° and (24.4 ± 0.2)°, when measured at a temperature in the range of from 20 to 30 °C with Cu-Kalpha_{1,2} radiation having a wavelength of 0.15419 nm.

6. The composition according to claim 4, wherein the other physical form of cabotegravir sodium is amorphous cabotegravir sodium.

7. A process for the preparation of the crystalline form of cabotegravir sodium as defined in any one of claims 1 to 3 (Form B) or the composition as defined in any one of claims 4 to 6 comprising:
(i) providing a crystalline form of cabotegravir sodium (Form A) **characterized by** having a PXRD comprising reflections at 2-Theta angles of (5.4 ± 0.2)°, (12.8 ± 0.2)°, (13.1 ± 0.2)°, (23.9 ± 0.2)° and (24.4 ± 0.2)°, when measured at a temperature in the range of from 20 to 30 °C with Cu-Kalpha_{1,2} radiation having a wavelength of 0.15419 nm.
(ii) slurrying the crystalline form of cabotegravir sodium (Form A) provided in step (i) in a solvent selected from the group consisting of cyclic ethers, C₃-C₄ ketones and methyl acetate or mixtures thereof, wherein the slurrying is for a time sufficient to effect transformation of cabotegravir sodium (Form A) to crystalline Form B of cabotegravir sodium of the present invention.

8. The process of claim 7, wherein the cyclic ethers are selected from 1,4-dioxane and tetrahydrofuran and the C₃-C₄ ketones are selected from acetone and 2-butanone.

9. The process of claim 7 or 8, further comprising step (iii) separating at least a part of the crystals obtained in step (ii) from the mother liquor.

10. The process of claim 9, further comprising step (iv) washing the isolated crystals obtained in step (iii).

11. The process according to any one of claims 7 to 10, further comprising step (v) drying the crystals obtained in any one of steps (ii) to (iv).

12. Use of the crystalline form as defined in any one of claims 1 to 3 or the composition as defined in any one of claims 4 to 6 for the preparation of a pharmaceutical composition.

13. A pharmaceutical composition comprising the crystalline form as defined in any one of claims 1 to 3 or the composition as defined in any one of claims 4 to 6, and at least one pharmaceutically acceptable excipient.

14. The pharmaceutical composition of claim 13, wherein the pharmaceutical composition is an oral solid dosage form, optionally a tablet or a capsule.

15. The pharmaceutical composition of claim 13 or 14 for use in the treatment and/or prophylaxis of viral infections, optionally viral infections caused by DNA viruses, RNA viruses, herpesviruses, retroviruses, hepadnaviruses, papillomavirus, hantavirus, adenoviruses and HIV.

## Patentansprüche

1. Kristalline Form von Cabotegravir-Natrium, **dadurch gekennzeichnet, dass** sie ein Pulver-Röntgendiffraktogramm aufweist, das Reflexionen bei 2-Theta-Winkeln von: (6,8 ± 0,2)°, (18,5 ± 0,2)° und (23,7 ± 0,2)°, gemessen bei einer Temperatur im Bereich von 20 bis 30 °C mit Cu-Kalpha_{1,2}-Strahlung mit einer Wellenlänge von 0,15419 nm, umfasst.

2. Kristalline Form nach Anspruch 1, ferner **gekennzeichnet dadurch, dass**
(i) es ein Pulver-Röntgendiffraktogramm, das Reflexionen bei 2-Theta-Winkeln von: (6,8 ± 0,2)°, (18,5 ± 0,2)°, (20,3 ± 0,2)° und (23,7 ± 0,2)°, gemessen bei einer Temperatur im Bereich von 20 bis 30 °C mit Cu-Kalpha_{1,2}-Strahlung mit einer Wellenlänge von 0,15419 nm umfasst, aufweist; oder
(ii) dass es ein Pulver-Röntgendiffraktogramm, das Reflexionen bei 2-Theta-Winkeln von: (6,8 ± 0,2)°, (18,5 ± 0,2)°, (18,9 ± 0,2)°, (20,3 ± 0,2)° und (23,7 ± 0,2)°, gemessen bei einer Temperatur im Bereich von 20 bis 30 °C mit Cu-Kalpha_{1,2} Strahlung mit einer Wellenlänge von 0,15419 nm umfasst, aufweist.

3. Kristalline Form nach Anspruch 1 oder Anspruch 2, **dadurch gekennzeichnet, dass** sie ein Pulver-Röntgendiffraktogramm aufweist, das das gleiche wie in Figur 1 der vorliegenden Erfindung ist, gemessen bei einer Temperatur im Bereich von 20 bis 30 °C mit Cu-Kalpha_{1,2}-Strahlung mit einer Wellenlänge von 0,15419 nm.

4. Zusammensetzung, umfassend die kristalline Form nach einem der Ansprüche 1 bis 3 und höchstens 20 Gew.-% einer anderen physikalischen Form von Cabotegravir-Natrium, bezogen auf das Gewicht der Zusammensetzung, vorzugsweise umfassend höchstens 10 Gew.-%, 5 Gew.-%, 2 Gew.-% oder 1 Gew.-% einer anderen physikalischen Form von Cabotegravir-Natrium, bezogen auf das Gewicht der Zusammensetzung.

5. Zusammensetzung nach Anspruch 4, wobei die andere physikalische Form von Cabotegravir-Natrium Form A ist, **gekennzeichnet durch** ein Pulver-Röntgendiffraktogramm, das Reflexionen bei 2-Theta-Winkeln von (5.4 ± 0,2)°, (12,8 ± 0,2)°, (13,1 ± 0,2)°, (23,9 ± 0,2)° und (24,4 ± 0,2)°, gemessen bei einer Temperatur im Bereich von 20 bis 30 °C mit Cu-Kalpha_{1,2} Strahlung mit einer Wellenlänge von 0,15419 nm, aufweist.

6. Zusammensetzung nach Anspruch 4, wobei die andere physikalische Form von Cabotegravir-Natrium amorphes Cabotegravir-Natrium ist.

7. Verfahren zur Herstellung der kristallinen Form von Cabotegravir-Natrium wie definiert in einem der Ansprüche 1 bis 3 (Form B) oder der Zusammensetzung wie in einem der Ansprüche 4 bis 6 definiert, umfassend:
(i) Bereitstellen einer kristallinen Form von Cabotegravir-Natrium (Form A), **dadurch gekennzeichnet, dass** sie eine PXRD aufweist, die Reflexionen bei 2-Theta-Winkeln von (5,4 ± 0,2)°, (12,8 ± 0,2)°, (13,1 ± 0,2)°, (23,9 ± 0,2)° und (24,4 ± 0,2)° umfasst, gemessen bei einer Temperatur im Bereich von 20 bis 30 °C mit Cu-Kalpha_{1,2}-Strahlung mit einer Wellenlänge von 0,15419 nm.
(ii) Aufschlämmen der kristallinen Form von Cabotegravir-Natrium (Form A), die in Schritt (i) zur Verfügung gestellt wurde, in einem Lösungsmittel, ausgewählt aus der Gruppe bestehend aus cyclischen Ethern, C₃-C₄-Ketonen und Methylacetat oder Mischungen davon, wobei das Aufschlämmen für eine Zeit erfolgt, die ausreichend ist, um die Umwandlung von Cabotegravir-Natrium (Form A) in die kristalline Form B von Cabotegravir-Natrium der vorliegenden Erfindung zu bewirken.

8. Verfahren nach Anspruch 7, wobei die cyclischen Ether ausgewählt sind aus 1,4-Dioxan und Tetrahydrofuran und die C₃-C₄-Ketone ausgewählt sind aus Aceton und 2-Butanon.

9. Verfahren nach Anspruch 7 oder 8, ferner umfassend Schritt (iii) das Trennen mindestens eines Teils der in Schritt (ii) erhaltenen Kristalle von der Mutterlauge.

10. Verfahren nach Anspruch 9, ferner umfassend Schritt (iv) Waschen der in Schritt (iii) erhaltenen isolierten Kristalle.

11. Verfahren nach einem der Ansprüche 7 bis 10, ferner umfassend Schritt (v) Trocknen der in einem der Schritte (ii) bis (iv) erhaltenen Kristalle.

12. Verwendung der kristallinen Form wie in irgendeinem der Ansprüche 1 bis 3 definiert, oder der Zusammensetzung wie in irgendeinem der Ansprüche 4 bis 6 definiert, zur Herstellung einer pharmazeutischen Zusammensetzung.

13. Pharmazeutische Zusammensetzung, umfassend die kristalline Form wie in irgendeinem der Ansprüche 1 bis 3 definiert, oder die Zusammensetzung wie in irgendeinem der Ansprüche 4 bis 6 definiert, und mindestens einen pharmazeutisch akzeptablen Hilfsstoff.

14. Pharmazeutische Zusammensetzung nach Anspruch 13, wobei die pharmazeutische Zusammensetzung eine orale feste Darreichungsform ist, optional eine Tablette oder eine Kapsel.

15. Pharmazeutische Zusammensetzung nach Anspruch 13 oder 14 zur Verwendung bei der Behandlung und/oder Prophylaxe von Virusinfektionen, optional Virusinfektionen verursacht durch DNA-Viren, RNA-Viren, Herpesviren, Retroviren, Hepadnaviren, Papillomaviren, Hantaviren, Adenoviren und HIV.

## Revendications

1. Forme cristalline du cabotégravir sodique **caractérisée en ce qu'**elle présente un diffractogramme de rayons X sur poudre comprenant des réflexions aux angles 2-Thêta de : (6,8 ± 0,2)°, (18,5 ± 0,2)° et (23,7 ± 0,2)°, quand la mesure est effectuée à une température située dans la plage allant de 20 à 30 °C avec un rayonnement Kalpha_{1,2} du Cu présentant une longueur d'onde de 0,15419 nm.

2. Forme cristalline selon la revendication 1, **caractérisé en outre**
(i) **en ce qu'**elle présente un diffractogramme de rayons X sur poudre comprenant des réflexions aux angles 2-Thêta de : (6,8 ± 0,2)°, (18,5 ± 0,2)°, (20,3 ± 0,2)° et (23,7 ± 0,2)°, quand la mesure est effectuée à une température située dans la plage allant de 20 à 30 °C avec un rayonnement Kalpha_{1,2} du Cu présentant une longueur d'onde de 0,15419 nm ; ou
(ii) **en ce qu'**elle présente un diffractogramme de rayons X sur poudre comprenant des réflexions aux angles 2-Thêta de : (6,8 ± 0,2)°, (18,5 ± 0,2)°, (18,9 ± 0,2)°, (20,3 ± 0,2)° et (23,7 ± 0,2)°, quand la mesure est effectuée à une température située dans la plage allant de 20 à 30 °C avec un rayonnement Kalpha_{1,2} du Cu présentant une longueur d'onde de 0,15419 nm.

3. Forme cristalline selon la revendication 1 ou la revendication 2, **caractérisé en ce qu'**elle présente un diffractogramme de rayons X sur poudre identique à celui représenté sur la Figure 1 de la présente invention, quand la mesure est effectuée à une température située dans la plage allant de 20 à 30 °C avec un rayonnement Kalpha_{1,2} du Cu présentant une longueur d'onde de 0,15419 nm.

4. Composition comprenant la forme cristalline selon l'une quelconque des revendications 1 à 3 et au plus 20 % en poids de n'importe quelle autre forme physique du cabotégravir sodique, sur la base du poids de la composition, de préférence comprenant au plus 10 % en poids, 5 % en poids, 2 % en poids, ou 1 % en poids de n'importe quelle autre forme physique du cabotégravir sodique, sur la base du poids de la composition.

5. Composition selon la revendication 4, dans laquelle l'autre forme physique du cabotégravir sodique est une Forme A **caractérisée en ce qu'**elle présente un diffractogramme de rayons X sur poudre comprenant des réflexions aux angles 2-Thêta de (5,4 ± 0,2)°, (12,8 ± 0,2)°, (13,1 ± 0,2)°, (23,9 ± 0,2)° et (24,4 ± 0,2)°, quand la mesure est effectuée à une température située dans la plage allant de 20 à 30 °C avec un rayonnement Kalpha_{1,2} du Cu présentant une longueur d'onde de 0,15419 nm.

6. Composition selon la revendication 4, dans laquelle l'autre forme physique du cabotégravir sodique est le cabotégravir sodique amorphe.

7. Procédé de préparation de la forme cristalline du cabotégravir sodique telle que définie dans l'une quelconque des revendications 1 à 3 (Forme B) ou de la composition telle que définie dans l'une quelconque des revendications 4 à 6, comprenant :
(i) la fourniture d'une forme cristalline du cabotégravir sodique (Forme A) **caractérisé en ce qu'**elle présente un PXRD comprenant des réflexions aux angles 2-Thêta de (5,4 ± 0,2)°, (12,8 ± 0,2)°, (13,1 ± 0,2)°, (23,9 ± 0,2)° et (24,4 ± 0,2)°, quand la mesure est effectuée à une température située dans la plage allant de 20 à 30 °C avec un rayonnement Kalpha_{1,2} du Cu présentant une longueur d'onde de 0,15419 nm.
(ii) la mise en suspension de la forme cristalline du cabotégravir sodique (Forme A) fournie dans l'étape (i) dans un solvant sélectionné dans le groupe consistant en les éthers cycliques, les cétones en C₃ à C₄ et l'acétate de méthyle ou les mélanges de ceux-ci, dans lequel la mise en suspension est pendant une durée suffisante pour effectuer la transformation du cabotégravir sodique (Forme A) en la Forme cristalline B du cabotégravir sodique de la présente invention.

8. Procédé selon la revendication 7, dans lequel les éthers cycliques sont sélectionnés parmi le 1,4-dioxane et le tétrahydrofurane et les cétones en C₃ à C₄ sont sélectionnées parmi l'acétone et la 2-butanone.

9. Procédé selon la revendication 7 ou 8, comprenant en outre une étape (iii) de séparation d'au moins une partie des cristaux obtenus dans l'étape (ii) à partir de la liqueur mère.

10. Procédé selon la revendication 9, comprenant en outre l'étape (iv) de lavage des cristaux isolés obtenus dans l'étape (iii).

11. Procédé selon l'une quelconque des revendications 7 à 10, comprenant en outre une étape (v) de séchage des cristaux obtenus dans l'une quelconque des étapes (ii) à (iv).

12. Utilisation de la forme cristalline telle que définie dans l'une quelconque des revendications 1 à 3 ou de la composition telle que définie dans l'une quelconque des revendications 4 à 6 pour la préparation d'une composition pharmaceutique.

13. Composition pharmaceutique comprenant la forme cristalline telle que définie dans l'une quelconque des revendications 1 à 3 ou la composition telle que définie dans l'une quelconque des revendications 4 à 6, et au moins un excipient pharmaceutiquement acceptable.

14. Composition pharmaceutique selon la revendication 13, dans laquelle la composition pharmaceutique est une forme galénique solide orale, facultativement un comprimé ou une capsule.

15. Composition pharmaceutique selon la revendication 13 ou 14 pour une utilisation dans le traitement et/ou la prophylaxie d'infections virales, facultativement des infections virales provoquées par des virus à ADN, des virus à ARN, des herpèsvirus, des rétrovirus, des hépadnavirus, un papillomavirus, un hantavirus, des adénovirus et un VIH.
